# EUROPÄISCHE PATENTSCHRIFT

(11) **EP 3 668 578 B1**
(45) Veröffentlichungstag und Bekanntmachung des Hinweises auf die Patenterteilung: **26.01.2022**
(21) Anmeldenummer: 18734133.4
(22) Anmeldetag: 15.06.2018
(51) Int. Cl.: A61M 25/00, A61M 1/36

(54) **DIALYSEKATHETER, INSBESONDERE ZUR LANGZEITANWENDUNG**
DIALYSIS CATHETER, IN PARTICULAR FOR LONG-TERM USE
CATHÉTER DE DIALYSE, EN PARTICULIER POUR UNE UTILISATION DE LONGUE DURÉE

(30) Priorität: 17.08.2017 DE 102017118819
(43) Veröffentlichungstag der Anmeldung: 24.06.2020
(73) Patentinhaber: Joline GmbH & Co. KG, 72379 Hechingen (DE)
(72) Erfinder: SEIDENBERGER, Dieter, 72379 Hechingen (DE)
(74) Vertreter: DREISS Patentanwälte PartG mbB
(86) Internationale Anmeldenummer: PCT/EP2018/065922
(87) Internationale Veröffentlichungsnummer: WO 2019/034301

(56) Entgegenhaltungen:
- WO-A1-2013/163172
- US-A1- 2008 154 186
- US-A1- 2013 253 445
- US-A1- 2015 088 100

## Beschreibung

Die Erfindung betrifft einen Dialysekatheter, insbesondere zur Langzeitanwendung, mit einem proximalen Ende zum Einführen in ein Blutgefäß, mit einem distalen Ende zum Anschluss an einen Dialysator, mit einer eine im Querschnitt runde Kontur aufweisenden Außenwandung, mit einer sich entlang einer Längsachse 1 erstreckenden, eine Mittelebene E_{w} bildende Mittelwandung, wobei die Mittelwandung ein Einleitlumen von einem Entnahmelumen trennt, und wobei das Entnahmelumen am proximalen Ende eine Entnahmeöffnung zum Entnehmen von Blut und das Einleitlumen am proximalen Ende eine Einleitöffnung zum Einleiten von gereinigtem Blut aufweist. Unter dem Begriff der Dialyse werden im Folgenden sämtliche Blutreinigungsverfahren verstanden, insbesondere die Hämodialyse, Hämofiltration, Hämodiafiltration, Peritonealdialyse, Hämoperfusion sowie Apherese-Verfahren.

Ein derartiger Katheter kann insbesondere einen längeren Zeitraum, mehrere Tage, Wochen oder Monate Verwendung finden. Der Katheter ist vorzugsweise aus einem Kunststoff und weiter vorzugsweise aus Polyurethan.

Derartige Katheter sind aus dem Stand der Technik in vielfältiger Art und Weise bekannt. Beispielsweise offenbart die EP 2 574 365 A1 sowie die EP 2 923 722 A1 derartige Katheter, die jeweils eine Mittelwandung aufweist, welche das Einleitlumen vom Entnahmelumen trennt.

Um ein geeignetes Entnehmen von Blut und um ein geeignetes Einleiten von gereinigtem Blut zu gewährleisten, weisen die proximalen Enden der bekannten Katheter verschiedene Geometrien auf. Insbesondere muss zum einen gewährleistet werden, dass ein Ansaugen der Einleitöffnung an einer Gefäßwand möglichst unterbunden wird. Zum anderen muss gewährleistet werden, dass das eingeleitete Blut nicht wieder durch die Entnahmeöffnung entnommen wird, um eine Rezirkulation zu unterbinden.

In WO 2013/163172 A1 sind Katheter offenbart, welche spreizbare Lumen am proximalen Ende aufweisen. In US 2015/0088100 A1 sind Katheter offenbart, welche seitlich spindelförmige Öffnungen am proximalen Ende aufweisen.

Der vorliegenden Erfindung liegt deshalb die Aufgabe zugrunde, einen Dialysekatheter vorzuschlagen, der ein geeignetes proximales Ende aufweist.

Diese Aufgabe wird dadurch gelöst, dass die Entnahmeöffnung in einer Ebene E₁ liegt, die die Mittelebene E_{W} in einer Schnittlinie s₁ unter einem spitzen Winkel w₁ schneidet, wobei die Schnittlinie s₁ und die Längsachse 1 einen spitzen Winkel α einschließen. Ferner ist vorgesehen, dass die Einleitöffnung in einer Ebene E₂ liegt, die die Mittelebene E_{W} in einer Schnittlinie s₂ unter einem spitzen Winkel w₂ schneidet, wobei die Schnittlinie s₁ und die Schnittlinie s₂ einen Winkel β einschließen, der im Bereich von 60° bis 120° liegt.

Dadurch, dass sowohl die Entnahmeöffnung als auch die Einleitöffnung nicht senkrecht zur Mittelwandung stehen, sondern mit der Mittelwandung jeweils einen spitzen Winkel w₁ und w₂ einschließen und dadurch, dass die Schnittlinien s₁ und s₂ selbst einen Winkel β einschließen, kann zum einen auf geeignete Weise Blut entnommen werden und zum anderen gereinigtes Blut in das Blutgefäß eingeleitet werden. Insbesondere dann, wenn der Winkel β im Bereich von 80° bis 100° liegt und weiter vorzugsweise im Bereich von 90° liegt, ergeben sich günstige Verhältnisse.

Der Winkel α kann vorzugsweise im Bereich von 30° bis 60° und vorzugsweise weiter im Bereich von 45° liegen. Auch hierdurch ergeben sich günstige Strömungsverhältnisse.

Ferner kann vorgesehen sein, dass die Winkel w₁ und w₂ identisch ausgebildet sein können. Die Winkel können dabei im Bereich von 30° bis 60° und weiter vorzugsweise im Bereich von 45° liegen.

Die Ausgestaltung kann dabei so sein, dass der Schnittpunkt S der Schnittlinie s₁ und der Schnittlinie s₂ außerhalb der Mittelwandung liegt. Dadurch ergibt sich ein geeigneter Abstand der Schnittlinie s₁ und der Schnittlinie s₂, wodurch wiederum günstige Verhältnisse geschaffen werden.

Das proximale Ende des Katheters weist vorzugsweise eine freie, von der Mittelwandung gebildete Endkante auf, die in einer Endlinie liegt. Diese Endlinie verläuft dabei vorzugsweise parallel zur Schnittlinie s₂ oder schließt mit der Schnittlinie s₂ einen Winkel ein, der im Bereich von 0° bis 15° liegt. In dem Fall, dass der Winkel 0° beträgt, liegt die Endlinie in der Schnittlinie s₂. Auch hieraus ergeben sich günstige Strömungsverhältnisse.

Die Mittelwandung als solche weist vorzugsweise parallel zur Längsachse 1 verlaufende Ränder auf, wobei die Endkante mit dem einen Rand einen spitzen Winkel w₄ und mit dem anderen Rand einen stumpfen Winkel w₅ einschließt. Die Endkante verläuft folglich schräg zur Längsachse 1.

Der Winkel w₄ kann dabei vorzugsweise im Bereich von 30° bis 60° und weiter vorzugsweise im Bereich von 45° liegen. Da die Ränder parallel zueinander verlaufen, weist der stumpfe Winkel w₅ einen Betrag auf, der 180° minus des Betrags von w₄ ist.

Es hat sich als vorteilhaft herausgestellt, wenn das Einleitlumen wenigstens eine von der Einleitöffnung beabstandet angeordnete Zusatzöffnung aufweist. Ferner hat sich als vorteilhaft herausgestellt, wenn das Entnahmelumen wenigstens eine von der Entnahmeöffnung beabstandet angeordnete Zusatzöffnung aufweist. Durch die Zusatzöffnungen kann zusätzlich Blut eingeleitet bzw. entnommen werden. Dies ist insbesondere dann erforderlich, wenn eine der Öffnungen an einem Blutgefäß zum Anliegen kommt und durch das Blutgefäß abgedeckt wird.

Vorteilhafterweise entspricht der Querschnitt des Einleitlumens dem Querschnitt des Entnahmelumens. Die beiden Lumen können spiegelsymmetrisch zur Mittelwandung ausgebildet sein.

Der erfindungsgemäße Katheter weist vorzugsweise zwei Lumen auf. Denkbar ist allerdings, dass auch ein drittes oder viertes Lumen vorgesehen sein kann, um beispielsweise Medikamente oder Spülflüssigkeiten in das Blutgefäß zu bringen.

Weitere Einzelheiten und vorteilhafte Ausgestaltungen der Erfindung sind der nachfolgenden Beschreibung zu entnehmen, anhand derer ein Ausführungsbeispiel der Erfindung näher beschrieben und erläutert ist.

Es zeigen:
- Figur 1: die Seitenansicht des proximalen Endes des Katheters mit der Entnahmeöffnung;
- Figur 2: die Ansicht gemäß Figur 1 um 180° um die Längsachse 1 gedreht;
- Figur 3: einen Querschnitt durch den Katheter gemäß Figur 1 entlang der Linie III;

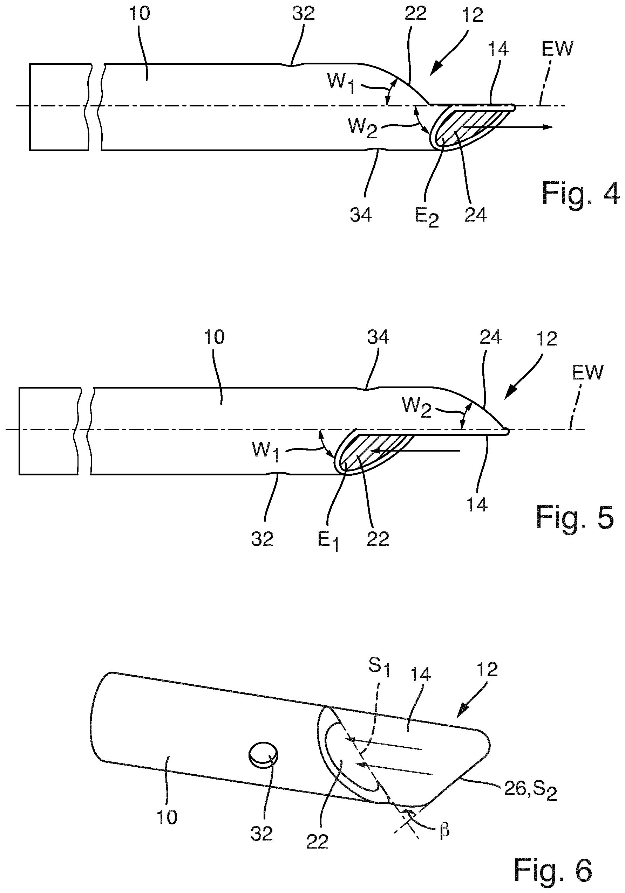

- Figur 4: die Unteransicht des proximalen Endes des Katheters gemäß Figur 1;
- Figur 5: die Draufsicht auf das proximale Ende des Katheters gemäß Figur 1; und
- Figur 6: eine perspektivische Ansicht des proximalen Endes des Katheters mit der Entnahmeöffnung.

In den Figuren ist ein Dialysekatheter 10 gezeigt, der ein proximales Ende 12 zum Einführen in ein Blutgefäß aufweist. Der Katheter 10 weist eine sich entlang einer Längsachse 1 erstreckende, eine Mittelebene E_{w} bildende Mittelwandung 14 auf.

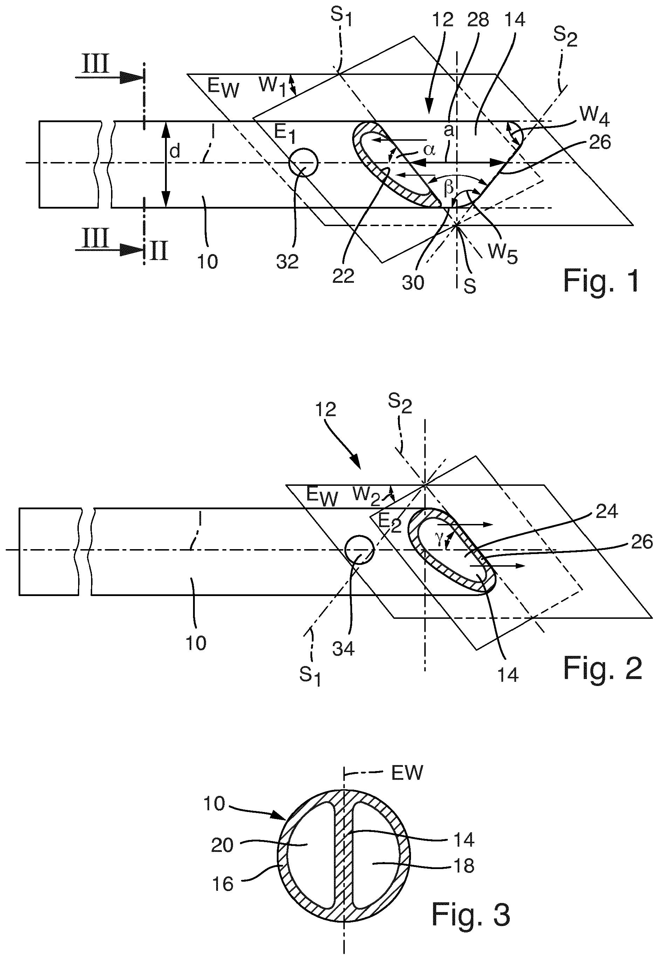

Wie der Figur 3 entnommen werden kann, weist der Katheter im Querschnitt eine runde Kontur mit einer Außenwandung 16 auf. Die Mittelwandung 16 trennt dabei das Innere des Katheters in ein Einleitlumen 18 und in ein Entnahmelumen 20. Am proximalen Ende 12 weist das Entnahmelumen 20 eine Entnahmeöffnung 22 auf. Die Entnahmeöffnung 22 liegt dabei in einer in Figur 1 angedeuteten Ebene E₁.

Das Einleitlumen 20 weist am proximalen Ende 12 eine Einleitöffnung 24 auf. Wie aus Figur 2 deutlich wird, liegt die Einleitöffnung 24 in einer Ebene E₂.

Die Ebene E₁ schließt dabei, wie in Figur 1 gezeigt ist, mit der Mittelebene E_{w} einen spitzen Winkel w₁ ein. Der Winkel w₁ weist dabei einen Betrag von ca. 45° auf. Wie ebenfalls aus Figur 1 deutlich wird, ist die Entnahmeöffnung 22 zum freien Ende des Katheters, der von einer freien Endkante 26 der Mittelwandung 14 gebildet wird, beabstandet angeordnet wird. Der Abstand a, gemessen entlang der Längsachse 1 entspricht dabei im Wesentlichen dem Durchmesser d der Außenwandung 16.

Wie aus Figur 2 deutlich wird, schließen die Mittelebene E_{w} und die Ebene E₂ den Winkel w₂ ein, wobei der Winkel w₂ im Bereich von 45° liegt. Wie ebenfalls aus Figur 2 deutlich wird, liegt die Endkante 26 in der Schnittlinie s₂ der Ebene E_{w} in der Ebene E₂. Die Schnittlinie s₂ schneidet dabei die Längsachse 1 in dem Winkel γ. Der Winkel γ beträgt vorzugsweise 45°.

Wie aus Figur 1 deutlich wird, schneidet die Ebene E₁ die Ebene E_{w} in der Schnittlinie s₁. Die Schnittlinie s₁ schneidet die Längsachse 1 unter dem Winkel α. Der Winkel α beträgt vorzugsweise 45°. Wie ebenfalls aus Figur 1 deutlich wird, schneidet die Schnittlinie s₁ die Schnittlinie S₂, die entlang der Endkante 26 verläuft, unter einem Winkel β. Der Winkel β liegt vorzugsweise im Bereich von 80° bis 90°.

Aus der Figur 1 wird zudem deutlich, dass der Schnittpunkt S der Schnittlinie s₁ mit der Schnittlinie s₂ außerhalb der Mittelwandung 14 liegt.

Zudem wird aus Figur 1 deutlich, dass die Mittelwandung 14 im Bereich des proximalen Endes 14, auf der der Entnahmeöffnung 22 proximalen Seite, zwei parallel zur Längsachse verlaufende Ränder 28, 30 aufweist. Der Rand 28 schließt dabei mit der Endkante 26 bzw. der Schnittlinie s₂ einen spitzen Winkel w₄ ein, der einen Betrag von 45° aufweist. Der andere Rand 30 schließt mit der Endkante 26 bzw. der Schnittlinie s₁ einen stumpfen Winkel w₅ ein, der im Bereich von 135° liegt.

Wie insbesondere in Figuren 1 und 2 deutlich wird, weist das Einleitlumen 20 von der Entnahmeöffnung 22 beabstandet eine Zusatzöffnung 32 auf. Diese Zusatzöffnung 32 dient dazu, dass Blut auch dann entnommen werden kann, falls die Einleitöffnung 22 verschlossen sein sollte, beispielsweise durch Anliegen an einem Blutgefäß. Entsprechend ist, wie aus Figur 2 deutlich wird, am Einleitlumen 18 eine Zusatzöffnung 34 vorgesehen.

Die beschriebene geometrische Ausgestaltung des proximalen Endes 12 des Katheters 10 hat den Vorteil, dass im Betrieb des Katheters 10 zum einen Blut optimal entnommen werden kann und zum anderen gereinigtes Blut optimal in ein Blutgefäß eingeleitet werden kann.

## Patentansprüche

1. Katheter (10) für die Dialyse, insbesondere zur Langzeitanwendung, mit einem proximalen Ende (12) zum Einführen in ein Blutgefäß, mit einer sich entlang einer Längsachsel erstreckenden, eine Mittelebene Ew bildende Mittelwandung (14), wobei die Mittelwandung (14) ein Einleitlumen (18) von einem Entnahmelumen (20) trennt, und wobei das Entnahmelumen (20) am proximalen Ende (12) eine Entnahmeöffnung (22) zum Entnehmen von Blut und das Einleitlumen (18) am proximalen Ende (12) eine Einleitöffnung (24) zum Einleiten von gereinigtem Blut aufweist, **dadurch gekennzeichnet, dass** die Entnahmeöffnung (22) in einer Ebene E₁ liegt, die die Mittelebene Ew in einer Schnittlinie siunter einem spitzen Winkel w₁ schneidet, wobei die Schnittlinie s₁ und die Längsachsel einen spitzen Winkel *a* einschließen, und dass die Einleitöffnung in einer Ebene E₂liegt, die die Mittelebene Ew in einer Schnittlinie s₂unter einem spitzen Winkel w₂schneidet, wobei die Schnittlinie s₁ und die Schnittlinie s₂ einen Winkel **ß** einschließen, der im Bereich von 60° bis 120° liegt.

2. Katheter (10) nach Anspruch 1, **dadurch gekennzeichnet, dass** der Winkel **ß** im Bereich von 80° bis 100° liegt und weiter vorzugsweise im Bereich von 90° liegt.

3. Katheter (10) nach Anspruch 1 oder 2, **dadurch gekennzeichnet, dass** der Winkel *a* im Bereich von 30° bis 60° und weiter vorzugsweise im Bereich von45° liegt.

4. Katheter (10) nach Anspruch 1, 2 oder 3, **dadurch gekennzeichnet, dass** die Winkel w₁ und w₂ gleich groß sind und/oder im Bereich von 30° bis 60° und weiter vorzugsweise im Bereich von 45° liegen.

5. Katheter (10) nach einem der vorhergehenden Ansprüche, **dadurch gekennzeichnet, dass** der Schnittpunkt S der Schnittlinie s₁ und der Schnittlinie s₂ außerhalb der Mittelwandung (14) liegt.

6. Katheter (10) nach einem der vorhergehenden Ansprüche, **dadurch gekennzeichnet, dass** das proximalen Ende (12) eine freie Endkante (26) aufweist, die in einer Endlinie liegt, wobei die Endlinie parallel zur Schnittlinie s₂ verläuft oder mit der Schnittlinie s₂ einen Winkel einschließen, der im Bereich von im 0° bis 15° liegt.

7. Katheter (10) nach Anspruch 6 **dadurch gekennzeichnet, dass** die Endkante (26) von der Mittelwandung (14) gebildet wird.

8. Katheter (10) nach Anspruch 6 oder 7, **dadurch gekennzeichnet, dass** die Mittelwandung (14) zwei zur Längsachsel parallel verlaufende Ränder (28, 30) aufweist, wobei die Endkante (26) mit dem einen Rand (28) einen spitzen Winkel w₄ und mit dem anderen Rand(30) einen stumpfen Winkel w₅einschließt.

9. Katheter (10) nach Anspruch 8, **dadurch gekennzeichnet, dass** der Winkel w₄ im Bereich von 30° bis 60° und weiter vorzugsweise im Bereich von 45° liegt.

10. Katheter (10) nach einem der vorhergehenden Ansprüche, **dadurch gekennzeichnet, dass** das Einleitlumen (18) wenigstens eine von der Einleitöffnung (22) beabstandet angeordnete Zusatzöffnung aufweist.

11. Katheter (10) nach einem der vorhergehenden Ansprüche, **dadurch gekennzeichnet, dass** das Entnahmelumen (20) wenigstens eine von der Entnahmeöffnung (22) beabstandet angeordnete Zusatzöffnung (32) aufweist.

12. Katheter (10) nach einem der vorhergehenden Ansprüche, **dadurch gekennzeichnet, dass** der Querschnitt des Einleitlumens (18) dem Querschnitt des Entnahmelumens (18) entspricht.

## Claims

1. Catheter (10) for dialysis, in particular for long-term use, having a proximal end (12) for insertion into a blood vessel, having a middle wall (14) extending along a longitudinal axis l and forming a midplane E_{w}, wherein the middle wall (14) separates an introduction lumen (18) from a removal lumen (20), and wherein the removal lumen (20) has at the proximal end (12) a removal opening (22) for the removal of blood, and the introduction lumen (18) has at the proximal end (12) an introduction opening (24) for the introduction of purified blood, **characterized in that** the removal opening (22) is located in a plane E₁ which intersects the midplane E_{w} in a section line s₁ at an acute angle w₁, wherein the section line s₁ and the longitudinal axis 1 enclose an acute angle α, and that the introduction opening is located in a plane E₂ which intersects the midplane E_{w} in a section line s₂ at an acute angle w₂, wherein the section line s₁ and the section line s₂ enclose an angle β which lies in the range from 60° to 120°.

2. Catheter (10) according to claim 1, **characterized in that** the angle β lies in the range from 80° to 100° and further preferably lies in the range of 90°.

3. Catheter (10) according to claim 1 or 2, **characterized in that** the angle α lies in the range from 30° to 60° and further preferably in the range of 45°.

4. Catheter (10) according to claim 1, 2, or 3, **characterized in that** the angles w₁ and w₂ are of the same size and/or lie in the range from 30° to 60° and further preferably in the range of 45°.

5. Catheter (10) according to one of the previous claims, **characterized in that** the intersection point S of the section line s₁ and the section line s₂ lies outside the middle wall (14).

6. Catheter (10) according to one of the previous claims, **characterized in that** the proximal end (12) has a free end edge (26) which is located in an end line, wherein the end line runs parallel to the section line s₂ or encloses an angle with the section line s₂, which lies in the range from 0° to 15°.

7. Catheter (10) according to claim 6, **characterized in that** the end edge (26) is formed by the middle wall (14) .

8. Catheter (10) according to claim 6 or 7, **characterized in that** the middle wall (14) has two edges (28, 30) running parallel to the longitudinal axis l, wherein the end edge (26) encloses an acute angle w₄ with one edge (28) and an obtuse angle w₅ with the other edge (30) .

9. Catheter (10) according to claim 8, **characterized in that** the angle w₄ lies in the range from 30° to 60° and further preferably in the range of 45°.

10. Catheter (10) according to one of the previous claims, **characterized in that** the introduction lumen (18) has at least one additional opening arranged at a distance from the introduction opening (22).

11. Catheter (10) according to one of the previous claims, **characterized in that** the removal lumen (20) has at least one additional opening (32) arranged at a distance from the removal opening (22).

12. Catheter (10) according to one of the previous claims, **characterized in that** the cross-section of the introduction lumen (18) corresponds to the cross-section of the removal lumen (18).

## Revendications

1. Cathéter (10) pour la dialyse, en particulier pour une utilisation de longue durée, avec une extrémité proximale (12) pour l'introduction dans un vaisseau sanguin, avec une paroi centrale (14) s'étendant le long d'un axe longitudinal l, formant un plan médian E_{w}, dans lequel la paroi centrale (14) sépare une lumière d'introduction (18) d'une lumière de prélèvement (20), et dans lequel la lumière de prélèvement (20) présente sur l'extrémité proximale (12) une ouverture de prélèvement (22) pour le prélèvement de sang et la lumière d'introduction (18) présente sur l'extrémité proximale (12) une ouverture d'introduction (24) pour l'introduction de sang purifié, **caractérisé en ce que** l'ouverture de prélèvement (22) se situe dans un plan E₁, qui croise le plan E_{W} dans une ligne d'intersection s₁ selon un angle w₁ aigu, dans lequel la ligne d'intersection s₁ et l'axe longitudinal l enferment un angle α aigu, et que l'ouverture d'introduction se situe dans un plan E₂, qui croise le plan médian E_{w} dans une ligne d'intersection s₂ selon un angle w₂ aigu, dans lequel la ligne d'intersection s₁ et la ligne d'intersection s₂ enferment un angle β, qui se situe dans la plage de 60° à 120°.

2. Cathéter (10) selon la revendication 1, **caractérisé en ce que** l'angle β se situe dans la plage de 80° à 100° et plus préférablement se situe dans la plage de 90°.

3. Cathéter (10) selon la revendication 1 ou 2, **caractérisé en ce que** l'angle α se situe dans la plage de 30° à 60° et plus préférablement dans la plage de 45°.

4. Cathéter (10) selon la revendication 1, 2 ou 3, **caractérisé en ce que** les angles w₁ et w₂ sont de même grandeur et/ou se situent dans la plage de 30° à 60° et plus préférablement dans la plage de 45°.

5. Cathéter (10) selon l'une quelconque des revendications précédentes, **caractérisé en ce que** le point d'intersection S de la ligne d'intersection s₁ et de la ligne d'intersection s₂ se situe à l'intérieur de la paroi centrale (14) .

6. Cathéter (10) selon l'une quelconque des revendications précédentes, **caractérisé en ce que** l'extrémité proximale (12) présente une arête d'extrémité (26) libre, qui se situe dans une ligne d'extrémité, dans lequel la ligne d'extrémité s'étend parallèlement à la ligne d'intersection s₂ ou enferme avec la ligne d'intersection s₂ un angle qui se situe dans la plage de 0° à 15°.

7. Cathéter (10) selon la revendication 6, **caractérisé en ce que** l'arête d'extrémité (26) est formée par la paroi centrale (14).

8. Cathéter (10) selon la revendication 6 ou 7, **caractérisé en ce que** la paroi centrale (14) présente deux bords (28, 30) s'étendant parallèlement à l'axe longitudinal l, dans lequel l'arête d'extrémité (26) enferme avec l'un des bords (28) un angle w₄ aigu et avec l'autre bord (30) un angle w₅ obtus.

9. Cathéter (10) selon la revendication 8, **caractérisé en ce que** l'angle w₄ se situe dans la plage de 30° à 60° et plus préférablement dans la plage de 45°.

10. Cathéter (10) selon l'une quelconque des revendications précédentes, **caractérisé en ce que** la lumière d'introduction (18) présente au moins une ouverture supplémentaire disposée à distance de l'ouverture d'introduction (22).

11. Cathéter (10) selon l'une quelconque des revendications précédentes, **caractérisé en ce que** la lumière de prélèvement (20) présente au moins une ouverture supplémentaire (32) disposée à distance de l'ouverture de prélèvement (22).

12. Cathéter (10) selon l'une quelconque des revendications précédentes, **caractérisé en ce que** la section transversale de la lumière d'introduction (18) correspond à la section transversale de la lumière de prélèvement (18).
